# EUROPEAN PATENT SPECIFICATION

(11) **EP 2 370 816 B1**
(45) Date of publication and mention of the grant of the patent: **30.04.2014**
(21) Application number: 09833528.4
(22) Date of filing: 17.12.2009
(51) Int. Cl.: G01N 33/68

(54) **METHOD FOR DIAGNOSIS OF CYSTIC FIBROSIS USING KL-6 LEVELS**
VERFAHREN ZUR DIAGNOSE VON ZYSTISCHER FIBROSE ANHAND DER KL-6-KONZENTRATION
PROCÉDÉ DE DIAGNOSTIC DE FIBROSE KYSTIQUE AU MOYEN DES NIVEAUX DE KL-6

(30) Priority: 18.12.2008 US 193717 P
(43) Date of publication of application: 05.10.2011
(73) Proprietor: EIDIA Co., Ltd., Tokyo 101-0032 (JP)
(72) Inventor: OHSHIMO, Shinichiro, Hiroshima-shi Hiroshima 734-8551 (JP); KOHNO, Nobuoki, Hiroshima-shi Hiroshima 734-8551 (JP); COSTABEL, Ulrich, Essen 45239 (DE)
(74) Representative: Woods, Geoffrey Corlett
(86) International application number: PCT/JP2009/071511
(87) International publication number: WO 2010/071228

(56) References cited:
- EP-A2- 0 200 464
- WO-A1-2004/106503
- WO-A2-2007/129114
- Q. A. AL-SALMI: "Serum KL-6 and Surfactant Proteins A and D in Pediatric Interstitial Lung Disease", CHEST, vol. 127, no. 1, 1 January 2005 (2005-01-01), pages 403-407, XP55033434, ISSN: 0012-3692, DOI: 10.1378/chest.127.1.403
- R. JANSSEN: "The Mucin-1 568 Adenosine to Guanine Polymorphism Influences Serum Krebs von den Lungen-6 levels", AMERICAN JOURNAL OF RESPIRATORY CELL AND MOLECULAR BIOLOGY, vol. 34, no. 4, 1 January 2006 (2006-01-01), pages 496-499, XP55033435, ISSN: 1044-1549, DOI: 10.1165/rcmb.2005-0151OC
- AKIHITO YOKOYAMA ET AL: "Prognostic value of circulating KL-6 in idiopathic pulmonary fibrosis", RESPIROLOGY, vol. 11, no. 2, 1 March 2006 (2006-03-01), pages 164-168, XP55033438, ISSN: 1323-7799, DOI: 10.1111/j.1440-1843.2006.00834.x
- RALPH C FRATES ET AL: "Infants and Young Children with Cystic Fibrosis Have High Levels of Serum Sialyl Lewisa Antigen", PEDIATRIC RESEARCH, vol. 37, no. 4, 1 April 1995 (1995-04-01), pages 460-464, XP55033440, ISSN: 0031-3998, DOI: 10.1203/00006450-199504000-00013
- VOYNOW, J.A., ET AL: "Mucin Gene Expression (MUC1, MUC2, and MUC5/5AC) in Nasal Epithelial Cells of Cystic Fibrosis, Allergic Rhinitis, and Normal Individuals", LUNG, vol. 176, 1998, pages 345-354, XP002680440,
- OHSHIMO S ET AL: "Serum KL-6 as a novel disease marker in adolescent and adult cystic fibrosis", SARCOIDOSIS VASCULITIS AND DIFFUSE LUNG DISEASES, PCA PUBLISHING, CORMANO, IT, vol. 26, no. 1, 1 July 2009 (2009-07-01), pages 47-53, XP009161314, ISSN: 1124-0490

## Description

### Technical Field

The present invention relates to a method for diagnosis of cystic fibrosis using KL-6 levels.

### Background Art

Cystic fibrosis (CF) is the most common life-shortening genetic disorder among Caucasian individuals, with an estimated frequency of 1:3,400 live births (for example, refer to Stat Med 1996; 15:449-462). The main pathogenetic factor of CF is a mutation in the cystic fibrosis transmembrane regulator gene (for example, refer to Science 1989; 245:1066-1073). Despite dramatic therapeutic advances, CF continues to be progressive, resulting in chronic pulmonary infection and shortened life expectancy. Previous studies have shown that impaired pulmonary function is the most sensitive prognostic factor for CF patients (for example, refer to Am J Respir Crit Care Med 2006; 173:659-666, Thorax 2001; 56:746-750, Jama 2001; 286:2683-2689, Eur Respir J 2000; 16:1056-1060, and N Engl J Med 1992; 326:1187-1191). Sensitive serum markers for predicting impaired pulmonary function and prognosis may be therefore useful to monitor disease progression. Although the specificity of a blood test is, in general, limited for evaluating the local pathology in the small airways of CF patients, it may still be beneficial because blood sampling is minimally invasive and can easily be repeated.

### DISCLOSURE OF INVENTION

The present invention has been made in view of the above-described circumstances. It provides a method for diagnosis of cystic fibrosis using a useful non-invasive maker.

According to a first aspect of the invention, there is provided a method for diagnosis of cystic fibrosis which comprising: measuring an amount of KL-6 in a sample of body fluid from a subject, and determining, based on the amount of KL-6 measured, the degree of presence of cystic fibrosis in the subject. It is favorable to measure the amount of KL-6 by an ELISA assay or an electrochemiluminescence immunoassay.

According to a second aspect of the invention, there is provided a method for determining the effectiveness of a treatment of cystic fibrosis in a subject, the method comprising: measuring an amount of KL-6 in a sample of body fluid from the subject, and determining the effectiveness of the treatment in the subject based on the amount of KL-6 measured.

### BRIEF DESCRIPTION OF THE DRAWINGS

Figure 1 shows a scatter plot graph showing a sex distribution of serum KL-6 levels in 80 healthy subjects.
Figure 2 shows a scatter plot graph showing an age distribution of serum KL-6 levels in 80 healthy subjects.
Figure 3 shows a scatter plot graph showing the distribution of serum KL-6 levels in patients with CF, healthy non-smoker subjects and healthy current smoker (Cu-smoker) subjects.
Figure 4 shows receiver operating characteristic curves for KL-6, LDH and CRP for the detection of cystic fibrosis.
Figure 5 shows scatter diagrams showing the correlation of serum KL-6 levels with VC % predicted.
Figure 6 shows scatter diagrams showing the correlation of serum KL-6 levels with FEV₁ % predicted.

### DESCRIPTION OF THE PREFERRED EMBODIMENT

The present invention relates to a method for diagnosis of cystic fibrosis using KL-6 levels. In particular, the invention relates to a method for evaluating the severity of cystic fibrosis after measuring the amount of KL-6 in a body fluid of a patient.

In the present invention, the term "step" is not only used for discrete step, but also for a step that is indistinguishable from other steps, so long as the intended purpose or results can be accomplished or obtained.

A first embodiment of the present invention provides a method for diagnosis of cystic fibrosis which comprising: measuring an amount of KL-6 in a sample of body fluid from a subject (measuring step); and determining, based on the amount of KL-6 measured, the degree of presence of cystic fibrosis in the subject (determining step). It is favorable to measure the amount of KL-6 by an ELISA assay or an electrochemiluminescence immunoassay.

KL-6 is a mucin-like glycoprotein with a molecular weight of 200 kd and has been classified as human MUC1 mucin (for example, refer to Int J Cancer Suppl 1994; 8:6-26). KL-6 has been reported to be a sensitive marker for various interstitial lung diseases, including idiopathic pulmonary fibrosis (IPF), radiation pneumonitis, drug-induced pneumonitis, collagen vascular disease-associated interstitial pneumonitis, extrinsic allergic alveolitis, pulmonary sarcoidosis and pulmonary alveolar proteinosis (for example, refer to Chest 1989; 96:68-73, Thorax 2003; 58:872-875, Chest 1996; 109:1276-1282, and Am J Respir Crit Care Med 1998; 158:1294-1298). In addition, prognostic significance of KL-6 has been also demonstrated in patients with IPF and drug-induced pneumonitis with diffuse alveolar damage or chronic interstitial pneumonia pattern (for example, Thorax 2003; 58:872-875 and Respirology 2006; 11:164-168). However, the significance of serum KL-6 in CF patients has not been investigated.

In the measuring step of the method of diagnosis of cystic fibrosis of the present invention, the amount of KL-6 in a sample of body fluid from a subject is measured.

The body fluid sample is preferably, but is not limited to, at least one selected from blood, plasma, lymphatic fluid and serum. The preparation of the sample of body fluid is not particularly limited and can be carried out by conventional methods.

The method for measuring the KL-6 is not limited so long as the amount of KL-6 in a sample of body fluid can be measured. Ordinary methods for detecting proteins may be used, such as a method using anti-KL-6 antibodies, ion exchange chromatography, mass spectrometry and the like. A measuring method using anti-KL-6 antibodies is preferable in view of detection sensitivity, detection specificity and convenience of detection. Anti-KL-6 antibodies are commercially available as a component of kits for enzyme-linked immunosorbent assay (ELISA) or electrochemiluminescence immunoassay (ECL) for detecting KL-6 such as Picolumi KL-6 or Eitest KL-6, manufactured by Sanko Junyaku, Tokyo, Japan.

In a preferred embodiment of the present invention, measurement of the KL-6 level may be performed by immunoassay including the use of anti-KL-6 antibodies and labeled antibodies.

The immunoassay may be carried out by any conventional assay without any particular limitation. Example of the immunoassay includes enzyme-linked immunosorbent assay (ELISA), chemiluminescence immunoassay, electrochemiluminescence immunoassay (ECL), absorption assay, a fluorescent antibody technique, radioimmunoassay (RIA), surface plasmon resonance, Western blotting, dot blotting and the like. In the present invention, enzyme-linked immunosorbent assay (ELISA) or electrochemiluminescence immunoassay (ECL) are preferable in view of detection sensitivity, detection specificity and convenience of detection. Kits for ELISA or ECL for detecting KL-6 are commercially available as, for example, Picolumi KL-6 or Eitest KL-6, manufactured by Sanko Junyaku, Tokyo, Japan.

Any known labeling agent ("a label") may used to label antibodies and materials in the embodiments of the present invention without any particular limitation. Examples of the label include enzymes, chemiluminescent materials, electrochemiluminescent materials, radioactive materials and the like. In the present invention, enzymes are preferable as a label in view of detection sensitivity and convenience of detection.

The enzymes may be used without any particular limitation so long as quantitative detection can be carried out by a physical or chemical procedure. Examples of the enzymes include alkaline phosphatase, horseradish peroxidase (HRP), luciferase and the like.

The method of detecting the labeled materials is not limited so long as the method is normally used for this purpose. Methods of detecting the labeled materials include, for example, absorbance spectrum, luminescence intensity, fluorescence intensity, or radiation measurement and the like. The method can be selected appropriately in accordance with the kind of label.

In a determining step of the present invention, the degree of presence of cystic fibrosis in the subject may be determined based on the amount of KL-6 measured.

For example, when the amount of KL-6 in the body fluid sample of the subject is equal to or higher than a reference level set in order to distinguish between a healthy person and a patient with cystic fibrosis, it can be determined that the subject has cystic fibrosis.

The reference level used in the determining step may be a level based on any criterion so long as it can used to distinguish between a healthy person and a patient with cystic fibrosis. A healthy person as a control is an individual who has been determined in advance not to have cystic fibrosis.

In a preferred embodiment of the present invention, the reference level may be a normal detection level (cut-off level) or an amount of KL-6 of a body fluid sample of another healthy person. A cut-off level can be determined as a value that is the average level detected in healthy persons plus a value of several times (for example, twice) the standard deviation. Furthermore, the cut-off level can be appropriately changed to another level in accordance with a sex, an age, a lifestyle factor such as smoking, ethnicity, the region in which patients live, and the like. For example, an amount of KL-6 of a healthy male person may be larger than that of a healthy female person, or an amount of KL-6 of a healthy elderly person may be larger than that of a healthy young person. The reference level may be determined according to at least a sex or an age of the subject.

The amount of KL-6 in the sample of body fluid from the subject may correlate with pulmonary function test variables. Pulmonary function test variables may change in according to the degree of severity of cystic fibrosis in the subject and the degree of severity of cystic fibrosis may be determined by the amount of KL-6.

For example, a vital capacity (VC) % and forced expiratory volume in one second (FEV₁) % may decrease according to the degree of disease severity of cystic fibrosis. On the other hand an increase in the measured amount of KL-6 may correlate with a decrease in VC and FEV₁. The degree of severity of cystic fibrosis may be determined based on the amount of KL-6 measured.

The second embodiment of the present invention is a method for determining the effectiveness of a treatment of cystic fibrosis in the subject, and the method comprises measuring an amount of KL-6 in a sample of body fluid from the subject (measuring step), and determining the effectiveness of the treatment in the subject based on the amount of KL-6 measured (determining step).

Matters relating to samples of body fluid and KL-6 with respect to the method of determining the effectiveness of the treatment of cystic fibrosis may be the same as in the description for the method for diagnosis of cystic fibrosis described above.

The measuring step of the method for determining the effectiveness of the treatment of cystic fibrosis can be carried out in the same manner as the measuring step of the method for diagnosis of cystic fibrosis described above.

In the determining step of the method for the determining the effectiveness of the treatment of cystic fibrosis, the effectiveness of the treatment of cystic fibrosis may be determined based on the amount of KL-6 in the sample of body fluid from the subject. The treatment is not particularly limited as long as it is a method of treatment of cystic fibrosis. By using the method for determining the effectiveness, the treatment of cystic fibrosis in the subject can be carried out more effectively.

For example, when the amount of KL-6 in the body fluid sample from the treated subject is equal to or lower than a reference level which is set according to the amount of KL-6 in the body fluid from the untreated subject, it can be determined that the treatment of cystic fibrosis may be effective on the patient. On the other hand, when the amount of KL-6 in the body fluid sample from the treated subject is higher than the reference level, it can be determined that the treatment of cystic fibrosis may not be effective. The reference level used in the determining step may be the amount of KL-6 in the untreated subject or a reference level which is selected based on the amount of KL-6 with acceptable error.

### EXAMPLES

In the following, the present invention will be described in more detail with reference to examples.

### <Study Subjects>

Consecutive patients with CF admitted to Ruhrlandklinik (Essen, Germany) for routine assessments between February and November 2007 were studied. The diagnosis was based on compatible clinical findings, genetic mutation or sweat test. At the enrollment, obvious interstitial lung diseases of known etiology, malignancy or an acute infection were excluded. Age- and sex-matched healthy subjects served as controls for serum KL-6 levels. All subjects were Caucasians. There were 72 patients with CF (all non-current smokers), 40 healthy non-smokers, and 40 healthy current smokers. No significant differences were observed in sex and age distribution between the groups (Table 1). The CF patients had a mean height of 170.0 ± 9.7 cm, a mean body weight of 59.5 ± 12.4 kg and a mean body mass index (BMI) of 20.5 ± 3.4. Serum samples were obtained from all subjects when taking routine blood tests and stored at - 80°C until analysis of KL-6. Documented informed consent was obtained from all subjects. The study was approved by the Institutional Review Board.

**Table 1**

| Factors | CF | Healthy (Non-smoker) | Healthy (Current smoker) |
|---|---|---|---|
| Number, n | 72 | 40 | 40 |
| Gender, Male/Female, n | 32/40 | 18/22 | 14/26 |
| Age (years), mean ± SD (range) | 30.6 ± 9.9 (15 - 63) | 32.6 ± 10.0 (16 - 58) | 35.5 ± 12.4 (19 - 64) |

### <KL-6 Assay>

Serum KL-6 level was measured by a sandwich enzyme-linked immunosorbent assay (ELISA) using the Eitest KL-6 ELISA kit (Eisai, Tokyo, Japan) according to the manufacturer's protocol. All samples were measured in duplicate, and mean values were used for subsequent analysis.

### <Pulmonary Function>

Vital capacity (VC) and forced expiratory volume in one second (FEV₁) were analyzed by using spirometry (ZAN 400 Sniff, ZAN Messgeraete GmbH, Germany). Values were expressed as percentages of predicted normal values.

### <Statistical Methods>

Data are expressed as mean ± standard deviation. Comparison of non-normally distributed variables between groups was done with Mann-Whitney U test. Correlations between serum KL-6 levels and pulmonary function valuables were analyzed with linear regression analysis. Fisher's LSD procedure was used for multiple comparison analysis. Paired t test was used to compare the areas under the ROC curve. All statistical analyses were done using SPSS version 13.0 for Windows (SPSS Inc., Chicago, IL). Differences were considered statistically significant when the p value was < 0.05.

### <Serum KL-6 Levels in Healthy Subjects>

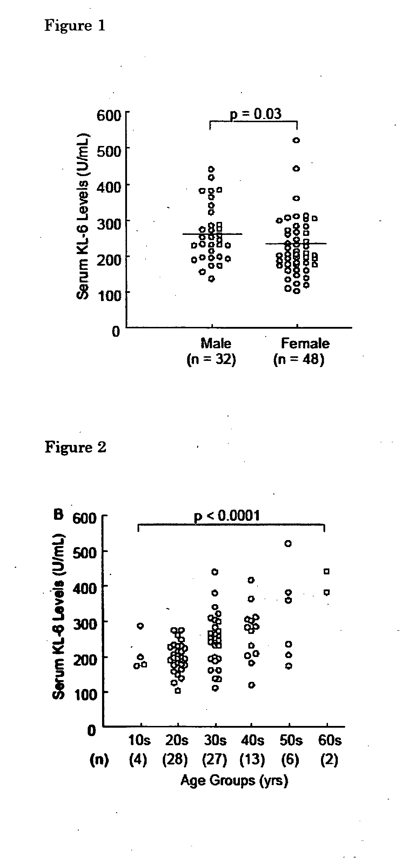

The distribution of serum KL-6 levels in 80 healthy subjects is shown in Figure 1 and Figure 2. In Figure 1 and Figure 2, bars indicate average. The mean serum KL-6 level was significantly higher in male subjects than in females (mean ± SD, male 262 ± 79 U/mL, female 225 ± 81 U/mL; p < 0.03). Serum KL-6 levels correlated with age (r = 0.45, p < 0.0001).

### <Serum KL-6 Levels in CF Patients>

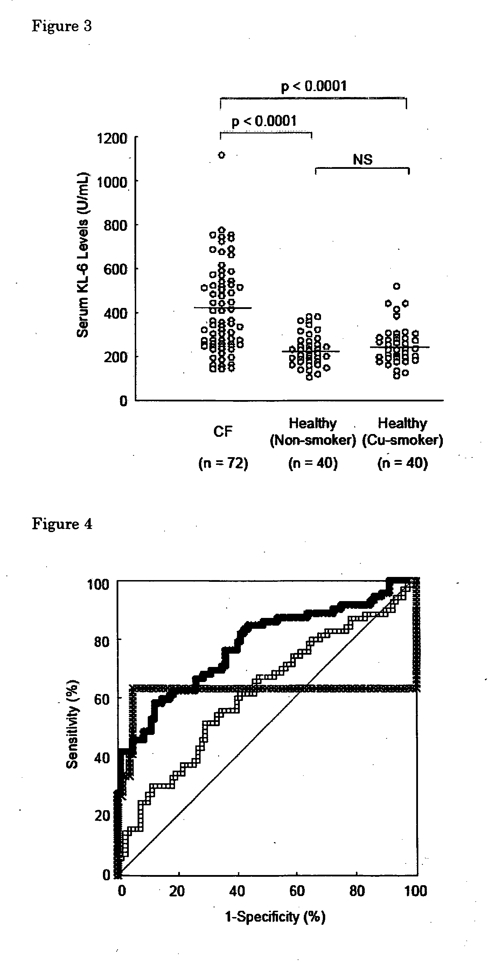

The mean serum KL-6 level was significantly elevated in CF patients compared with healthy subjects (mean ± SD, CF 410 ± 200 U/mL, healthy non-smokers 228 ± 73 U/mL, healthy current smokers 252 ± 90 U/mL, p < 0.0001, p < 0.0001, respectively) (Figure 3). In Figure 3, bars indicate average values and NS means "Not statistically significant". After adjustment for age and sex, the differences between groups were still significant. There was no significant difference in serum KL-6 levels between healthy non-smokers and healthy current smokers. Site of involved organ(s), presence of chronic infection(s), use of inhalation therapy, use of oral medication and frequency of hospitalization had no significant effect on serum KL-6 levels (data not shown).

### <Receiver Operating Characteristic (ROC) Curve Analysis>

ROC curve analysis was used to evaluate the sensitivity, specificity and diagnostic accuracy of serum KL-6, LDH and CRP levels (Figure 4). In Figure 4, closed circles indicate KL-6, opened squares indicate LDH and crosses indicate CRP. The largest area under the curve (AUC) was found for KL-6: KL-6, 0.79 (95% confidence interval (CI), 0.71 to 0.86); LDH, 0.62 (95% CI, 0.53 to 0.71); CRP, 0.71 (95% CI, 0.62 to 0.81). When the cut-off levels were set as the closest point to 100% sensitivity and 100% specificity, the levels were 310 U/mL for KL-6 (sensitivity, 63%; specificity, 85%; and diagnostic accuracy, 74%, respectively), 195 IU/L for LDH (51%; 71%; and 61%, respectively), and 0.6 mg/dl for CRP (58%; 95%; and 77%, respectively). The differences in the AUC between KL-6, LDH and CRP was significant (KL-6 and LDH, p = 0.0005; KL-6 and CRP, p = 0.0002, LDH and CRP, p = 0.001).

### <Correlation of Serum KL-6 Levels with Pulmonary Function Test Variables>

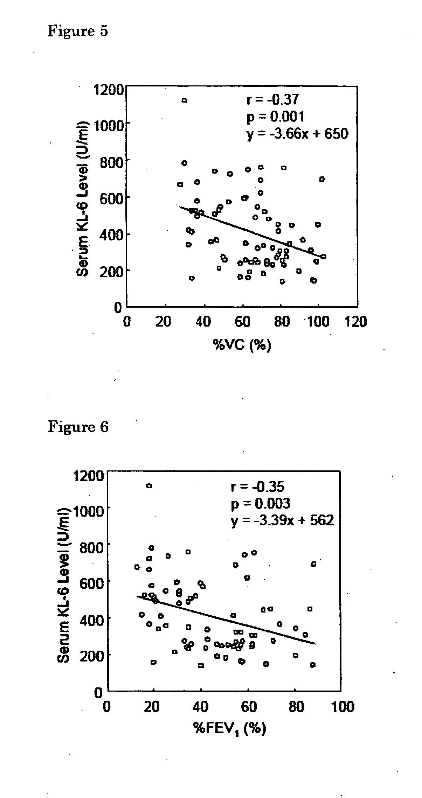

Serum KL-6 levels showed an inverse relationship with VC % predicted (r = -0.37, p = 0.001) (Figure 5), and with FEV₁ % predicted (r = -0.35, p = 0.003) (Figure 6). In Figure 5 and Figure 6, the r value indicates a correlation coefficient, the equation indicates a regression equation, and lines indicate regression lines. When the 72 CF patients were categorized into two groups based on the values of VC % predicted (above or equal to 40%, n = 59; under 40%, n = 13) or FEV1 % predicted (above or equal to 40%, n = 40; under 40%, n = 32), patients with the lower VC or FEV1 showed significantly higher serum KL-6 levels (p = 0.01, p = 0.0009, respectively).

This study is the first that explored the utility of KL-6 as a serum marker in patients with CF. Serum KL-6 levels in CF patients were significantly increased compared to healthy control subjects. After adjustment for age and sex the difference was still significant. Increased serum KL-6 levels correlated with lower values of FEV₁ and VC. Serum KL-6 levels in CF patients were not influenced by extrapulmonary lesions, presence of chronic infection or mode of therapy. Interestingly, compared with traditional markers of cell injury or inflammation such as LDH and CRP, serum KL-6 discriminated best between CF patients and healthy controls.

We have previously reported that serum KL-6 levels are increased in various interstitial lung diseases (for example, refer to Chest 1989; 96:68-73, Thorax 2003; 58:872-875, Chest 1996; 109:1276-1282, and Am J Respir Crit Care Med 1998; 158:1294-1298). The significant correlation of serum KL-6 level with pulmonary function tests indicates that serum KL-6 is a non-invasive marker for evaluating the degree of lung destruction in CF patients.

FEV₁ is an important prognostic factor in CF patients (for example, refer to N Engl J Med 1992; 326:1187-1191 and Thorax 1997; 52:291-293). Kerem et al. have demonstrated that FEV₁ <30% predicted was associated with a mortality rate of 50% within 2 years (for example, refer to N Engl J Med 1992; 326:1187-1191). Milla et al. have shown that the yearly decline in FEV₁ was predictive of mortality in CF (for example, refer to Chest 1998; 113:1230-1234). In addition, CF patients with a low FEV₁ are more likely to experience pulmonary exacerbations (for example, refer to Thorax 2006; 61:969-974). The inverse correlation of serum KL-6 with FEV₁ indicates the ability of KL-6 to assess the severity of airway remodeling due to fibrosis in CF. The local concentrations of KL-6 in BALF, sputum or exhaled breath condensate are also good biomarker for CF as serum levels. In addition these findings can be applied to children.

Only few studies have shown that a serum marker may be useful for predicting decline in lung function in CF patients. McColley et al. have demonstrated that the serum level of vascular endothelial growth factor (VEGF) was increased compared to healthy subjects and predicted the decrease in FEV₁ (for example, refer to Am J Respir Crit Care Med 2000; 161:1877-1880). Carlsson et al. have shown that an increased serum level of autoantibodies against bactericidal permeability increasing protein (BPI-ANCA) predicted a decrease in FEV₁ and a poor prognosis (for example, refer to J Cyst Fibros 2007; 6:228-233). Other investigators studied local concentrations of potential biomarkers in CF patients. Meyer et al. reported that active neutrophil elastase (NE) levels and active myeloperoxidase levels in BALF correlated inversely with FEV₁ (for example, refer to J Lab Clin Med 1993; 121:654-661). O'Connor et al. demonstrated that active NE levels in sputum correlated with the severity of pulmonary disease; they also found that the levels of α1-proteinase inhibitor (α1-PI), a NE inhibitor, were increased both in sputum and plasma compared with control subjects (for example, refer to Am Rev Respir Dis 1993; 148:1665-1670).

Moreover, several proinflammatory cytokines including tumor necrosis factor (TNF)-α, interleukin (IL)-1, IL-6 and IL-8 have been found to be increased in BALF or sputum of CF patients (for example, refer to Am J Respir Crit Care Med 1995; 152:2111-2118, Am J Physiol Lung Cell Mol Physiol 2000; 278:L33-41, and Eur Respir J 1999; 14:339-346). These proinflammatory markers could potentially be useful for evaluating CF patients. Wolter et al., however, have demonstrated a divergence between the local and systemic inflammatory response (for example, refer to Clin Diagn Lab Immunol 1999; 6:260-265). IL-8 and TNF-α were generally undetectable in serum. No or only weak correlations were found between serum and sputum levels of proinflammatory markers, including IL-8, TNF-α, α1-PI and NE-α1-PI complex (for example, refer to Clin Diagn Lab Immunol 1999; 6:260-265).

The predominant histological feature of the lung in CF is an inflammation of the small airways with resultant obstruction and surrounding fibrosis. Serum KL-6 levels are known to be increased in various fibrotic lung diseases (for example, refer to Chest 1989; 96:68-73, Thorax 2003; 58:872-875, Chest 1996; 109:1276-1282, and Am J Respir Crit Care Med 1998; 158:1294-1298). The increase of serum KL-6 levels in interstitial pneumonitis is thought to be due to an enhanced production of KL-6 by regenerating alveolar type II pneumocytes, and/or due to an increased permeability following destruction of the air-blood barrier in the affected lungs (for example, refer to Am Rev Respir Dis 1993; 148:637-642 and Am J Respir Crit Care Med 1997; 156:109-115). The concentration of KL-6 in BALF corresponds well with serum KL-6 in patients with interstitial lung diseases (for example, refer to Am Rev Respir Dis 1993; 148:637-642). We have previously demonstrated that the purified KL-6 molecule has chemotactic, proliferative and anti-apoptotic effects on fibroblasts in vitro, and that the proliferative and anti-apoptotic effects of KL-6 are additive to those of transforming growth factor-β (for example, refer to Biochem Biophys Res Commun 2005; 338:1845-1852 and Am J Respir Cell Mol Biol 1997; 17:501-507). The increase of serum KL-6 in CF patients is likely to favor accelerated fibrosis.

Stahel et al. have immunohistochemically demonstrated that the location of KL-6 is in the epithelial cells of the pancreatic and mammary ducts, as well as in the type II epithelial cells of the lung (for example, refer to Int J Cancer Suppl 1994; 8:6-26). Sakuma et al. have shown that serum KL-6 levels decreased by 36% following lobectomy, indicating that circulating KL-6 is predominantly derived from the lung (for example, refer to Surg Today 1999; 29:121-128). Our study demonstrated no significant correlation between serum KL-6 levels and the presence of pancreas insufficiency or glucose tolerance, indicating that KL-6 is a lung-specific marker in CF patients.

Our study is the first to show the distribution of serum KL-6 levels in healthy Caucasian subjects. In these subjects, serum KL-6 levels are higher in males than in females and show a correlation with age, whereas there was no difference between healthy smokers and non-smokers. These findings are compatible with a previous study performed in Japanese subjects (for example, refer to Jpn J Pathol 1996; 44:653-658).

In conclusion, we demonstrated increased serum KL-6 levels in CF patients and an inverse correlation with FEV₁ and VC. These findings indicate that serum KL-6 is a useful marker to assess the disease severity.

## Claims

1. A method for diagnosis of cystic fibrosis, the method comprising:
measuring an amount of KL-6 in a sample of body fluid from a subject, and
determining, based on the amount of KL-6 measured, the presence or absence of cystic fibrosis in the subject.

2. The method according to claim 1, wherein the amount of KL-6 is measured by an ELISA assay or an electrochemiluminescerice immunoassay.

3. The method according to claim 1, which further comprises determining the degree of severity of cystic fibrosis in the subject based on the amount of KL-6 measured.

4. A method for determining the effectiveness of a treatment of cystic fibrosis in a subject, the method comprising:
measuring an amount of KL-6 in a sample of body fluid from the treated subject, and
determining the effectiveness of the treatment in the subject based on the amount of KL-6 measured.

## Patentansprüche

1. Verfahren zur Diagnose von zystischer Fibrose, wobei das Verfahren umfasst:
Messen der Menge an KL-6 in einer Probe Körperflüssigkeit eines Probanden und Bestimmung auf der Grundlage der gemessenen Menge an KL-6, ob zystische Fibrose in dem Probanden vorliegt oder nicht.

2. Verfahren nach Anspruch 1, wobei die Menge an KL-6 durch einen ELISA-Assay oder einen Elektrochemilumineszenz-Immunoassay gemessen wird.

3. Verfahren nach Anspruch 1, ferner umfassend die Bestimmung des Schweregrads der zystischen Fibrose in dem Probanden auf der Grundlage der gemessenen Menge an KL-6.

4. Verfahren zur Bestimmung der Wirksamkeit einer Behandlung von zystischer Fibrose in einem Probanden, wobei das Verfahren umfasst:
Messen einer Menge an KL-6 in einer Probe Körperflüssigkeit des behandelnden Probanden und Bestimmung der Wirksamkeit der Behandlung in dem Probanden auf der Grundlage der gemessenen Menge an KL-6.

## Revendications

1. Procédé pour diagnostiquer la fibrose kystique, le procédé comprenant :
la mesure d'une quantité de KL-6 dans un échantillon de liquide biologique provenant d'un sujet, et
la détermination, sur la base de la quantité de KL-6 mesurée, de la présence ou de l'absence de fibrose kystique chez le sujet.

2. Procédé selon la revendication 1, où la quantité de KL-6 est mesurée par un test ELISA ou un immunotest à électrochimiluminescence.

3. Procédé selon la revendication 1, qui comprend en outre la détermination du degré de gravité de fibrose kystique chez le sujet sur la base de la quantité de KL-6 mesurée.

4. Procédé pour déterminer l'efficacité d'un traitement de fibrose kystique chez un sujet, le procédé comprenant :
la mesure d'une quantité de KL-6 dans un échantillon de liquide biologique provenant du sujet traité, et
la détermination de l'efficacité du traitement chez le sujet sur la base de la quantité de KL-6 mesurée.
